# EUROPEAN PATENT SPECIFICATION

(11) **EP 3 202 350 B1**
(45) Date of publication and mention of the grant of the patent: **02.06.2021**
(21) Application number: 15847084.9
(22) Date of filing: 13.07.2015
(51) Int. Cl.: A61B 18/14

(54) **ENERGY DEVICE FOR SURGICAL OPERATIONS**
ENERGIEVORRICHTUNG FÜR CHIRURGISCHE EINGRIFFE
DISPOSITIF ÉNERGÉTIQUE POUR OPÉRATIONS CHIRURGICALES

(30) Priority: 30.09.2014 JP 2014201277
(43) Date of publication of application: 09.08.2017
(73) Proprietor: Tocalo Co., Ltd., Kobe-shi, Hyogo 658-0013 (JP); National University Corporation Shiga University of Medical Science, Otsu-shi Shiga 520-2192 (JP)
(72) Inventor: MIKI, Shinya, Akashi-shi Hyogo 674-0093 (JP); ADACHI, Megumi, Akashi-shi Hyogo 674-0093 (JP); TANI, Toru, Otsu-shi Shiga 520-2192 (JP); NAKA, Shigeyuki, Otsu-shi Shiga 520-2192 (JP); SHIMIZU, Tomoharu, Otsu-shi Shiga 520-2192 (JP)
(74) Representative: Grünecker Patent- und Rechtsanwälte PartG mbB
(86) International application number: PCT/JP2015/070066
(87) International publication number: WO 2016/051918

(56) References cited:
- EP-A1- 2 298 198
- WO-A1-97/11649
- WO-A2-2005/046739
- JP-A- H11 508 171
- JP-A- 2007 510 519
- JP-A- 2010 284 439
- US-A- 5 549 604
- US-A1- 2006 259 033
- US-A1- 2008 027 428
- US-B1- 6 409 725

## Description

### TECHNICAL FIELD

This invention relates to an energy device for surgical operation provided with a function capable of performing incision of biological tissue, coagulation and hemostasis by acting energy through microwave, radio-frequency wave or ultrasonic wave represented by heat to a target portion of a body.

### RELATED ART

In general, the energy device for surgical operation is used for transmitting microwave, radio-frequency wave or ultrasonic wave from an operational area portion to a target portion of a body to perform incision of biological tissue, bleed coagulation from the tissue (hemostasis) or both of incision and coagulation by these actions. In the energy device for surgical operation, biological tissue is fixed to the operational area portion in the treatment to the biological tissue, whereby an impedance, discharging property, electric conductivity and heat conductivity of an electrode and transmission characteristics of energy are changed or smoke is emitted or treating properties such as cutting, coagulating performance, stop bleeding and the like may be deteriorated. As a countermeasure to such problems, it is known to form a coating layer hardly fixing the biological tissue to the operational area portion. The conventional art will be described with reference to documents 1-4 below.

In Patent Document 1 is described that the conventional energy device for surgical operation is constructed with a portion made of a metal and having an electric conductivity obtained by forming irregularities on a surface of an operational area portion made of a metal, filling a resin having a low surface energy such as fluorine resin or the like in concave portions and then making the surface relatively smooth, and a portion made of a low surface energy resin and having an insulation property and a low surface energy. In the energy device for surgical operation of such a construction, a fluorine polymer embedded in the irregularities on the surface of the operational area portion is partly exposed to reduce fixation of biological tissue.

In Patent Document 2 is disclosed that at least a part of the surface of the operational area portion made of a metal in the conventional energy device for surgical operation is constructed with a thermoplastic fluorine resin. In the energy device for surgical operation of such a construction, the thermoplastic fluorine resin disposed in the surface of the operational area portion is strongly pushed onto biological tissue, while an exposed portion of the metal is weakly pushed thereon, whereby the fixation of the biological tissue to the operational area portion is reduced.

In the conventional energy device for surgical operation of Patent Document 3, the surface of the operational area portion made of a metal and coarsened by blast treatment is constructed with a primer layer containing polysiloxane resin and an outermost coating of fluorine polymer made of silicone elastomer based polymer. The outermost coating is non-uniform in the thickness and an edge part of a blade for surgical operation is thin and has a thickness of 76-510 µm on its main surface. In the energy device for surgical operation of such a construction, high-frequency wave is preferentially conducted in the edge part having a thin coating to incise and coagulate biological tissue, while the main surface not conducting the high-frequency wave is free of heat affection and hence the fixation of biological tissue is reduced.

In the conventional energy device for surgical operation of Patent Document 4, the surface of the operational area portion made of stainless steel is constructed with an oxide layer oxidized by heat treatment and a releasing material containing silicone. In the energy device for surgical operation of such a construction, the coating with non-uniform thickness is obtained to reduce the fixation of biological tissue similarly in Patent Document 3 and at the same time the adhesion property is improved by the formation of the oxide layer.

### PRIOR ART DOCUMENTS

### PATENT DOCUMENTS

Patent Document 1: JP-A-H10-500051
Patent Document 2: JP-A-2011-72324
Patent Document 3: Japanese Patent No. 3182153
Patent Document 4: Japanese Patent No. 4502565

WO 97/11649 A1, US 2006/259033 A1, EP 2 298 198 A1, WO 2005/046739 A2 and US 2008/027428 A1 disclose further relevant prior art.

### SUMMARY OF THE INVENTION

### TASK TO BE SOLVED BY THE INVENTION

In the energy device for surgical operation disclosed in Patent Document 1, since the fluorine polymer is merely embedded in the concave portions of the surface for preventing the fixation of biological tissue, there is a fear of dropout. Also, the convex portions having an electric conductivity is exposed even when the fluorine polymer is disposed partly, so that the effect of sufficiently preventing the fixation cannot be developed. Furthermore, the document describes a step of subjecting an electrode support to coarsening by blasting or the like or mechanical deformation surface finishing by buffing or the like, and suggests that the step cannot be applied to an electrode support of a complicated form.

In the energy device for surgical operation described in Patent Document 2, the thermoplastic fluorine resin for preventing the fixation of biological tissue is arranged in at least a part of the operational area portion in form of line or dot or lattice, but the base material of a metal having an electric conductivity is exposed, so that the effect of sufficiently preventing the fixation cannot be developed. Also, the formation of the thermoplastic fluorine resin is performed by heating and pressing at 1-4 MPa and 320-340°C for 1-30 minutes, so that there is a fear of deforming the base material and there is an issue that it cannot be applied to the complicated form.

In the energy device for surgical operation described in Patent Document 3, the primer layer containing polysiloxane resin and the outermost coating of fluorine polymer made of silicone elastomer based polymer are applied for preventing the fixation of biological tissue. Since the outermost coating is formed by an immersion treatment, the thickness becomes thin in the edge part of the blade and thick in the flat part thereof. By utilizing this feature is introduced energy into the edge part having a thin thickness, whereby the incision and coagulation of biological tissue can be performed, while the flat part having a thick thickness is insulated thermally, whereby the fixation of biological tissue can be prevented. In this method, however, the thickness is dependent on the form of the operational area portion, so that the introduction part cannot be designed arbitrarily. Since the adhesion force of the primer layer containing polysiloxane resin to the base material is not high, a step of performing sand blasting with aluminum oxide media is described in a working example of the production method as a countermeasure, which cannot be applied to a complicated form.

In the energy device for surgical operation described in Patent Document 4, the releasing material containing silicone is applied to the surface of the operational area portion made of stainless steel for preventing the fixation of biological tissue. As a method of adhering the releasing material containing silicone is described that an iron oxide layer having a highest adhesion force is formed on a ground material before the coating of silicone by subjecting the surface of the operational area portion in the base material of stainless steel to a heat treatment at 750°F for 30 minutes in the examples without requiring a middle layer or a coarsened layer. However, this method has an issue that the base material is deformed or rusting is easily caused by sensitization. Also, the base material is limited to an iron based material.

The invention is made for solving the above issues of the prior art and an object thereof is to provide an energy device for surgical operation capable of preventing the fixation of biological tissue by forming a coating layer having a good adhesiveness and applicable to a complicated form.

### SOLUTION FOR TASK

In order to achieve the above object, the invention is an energy device for surgical operation having an operational area portion for transmitting energy to a body target portion, as disclosed in appended independent claim 1. Preferred embodiments are disclosed in the dependent claims.

In the energy device for surgical operation of the above construction, the fixation of biological tissue to the operational area portion is suppressed by water-repellent and oil-repellent actions of the outermost coating having a low surface energy. In the energy device for surgical operation of the above construction, good adhesiveness is obtained by strong adhesion of the base coating to the base material and the outermost coating. Further, the base coating can be formed at room temperature or about 150°C, so that the deformation of the base material due to heat affection can be suppressed.

As a preferable embodiment of the invention, a surface roughness of the base material constituting the operational area portion is not more than 3 µm as Ra value before the formation of the coating layer, preferably 0.01-1.0 µm. In the energy device for surgical operation of this construction, the surface can be adjusted arbitrarily by electrolytic polishing, buffing, blast treatment with spherical or polygonal ceramic abrasive grains or laser irradiation to select the surface form and roughness effective for suppressing the fixation of biological tissue.

The invention is further defined in that the base coating has a thickness of 0.1-1.0 µm. In the energy device for surgical operation of this construction, the base coating is a thin coating, so that the formation of uniform coating can be attained even in the base material of a complicated form. The fixation of biological tissue can be prevented when the thickness of the outermost coating comprised of polysiloxane or a compound containing polysiloxane or a compound containing a partly fluorinated polysiloxane is not less than 0.1 µm.

Moreover, the base material constituting the operational area portion is a metal, preferably stainless steel, titanium or a titanium alloy, or a resin, preferably polyimide or PEEK resin. In the energy device for surgical operation of this construction, the above base material can be generally taken as a material having a biological safety and provides good adhesiveness to the base coating. Also, the base material constituting the operational area portion is applicable to portions other than the base material and is not limited.

As another preferable embodiment of the invention, energy transmitted from the operational area portion to biological tissue is microwave, radio-frequency wave or ultrasonic wave. In the energy device for surgical operation of this construction, the fixation of body tissue can be suppressed by applying the above coating even in any energy used in the incision of body tissue, coagulation or stop bleeding.

As the other preferable embodiment of the invention, the energy device for surgical operation is a surgical knife, tweezers, forceps or snare used in surgical operation. The energy device for surgical operation of this construction can be applied as surgical knife, tweezers, forceps or snare particularly used in the surgical operation by uniformly coating the whole periphery of the operational area portion with the coating layer for suppressing the fixation of body tissue.

### EFFECT OF THE INVENTION

As mentioned above, the invention can provide an energy device for surgical operation capable of suppressing fixation of body tissue in an operational area portion of the energy device for surgical operation.

### BRIEF DESCRIPTION OF THE DRAWINGS

FIGS. 1(a) and (b) are diagrams illustrating a construction of an electrosurgical knife as an example of the energy device for surgical operation according to the invention, respectively.
FIG. 2 is a sectional view showing a coating layer in a test specimen.
FIG. 3 is a photomicrograph showing results obtained when a base coating made of silicon oxide and an outermost coating made of a compound containing fluorinated polysiloxane are formed on a base material of an operational area portion in a surgical knife, tweezers, forceps or snare and the sectional structure of each coating is observed by an electron microscope in close-up.

### EMBODIMENTS FOR CARRYING OUT THE INVENTION

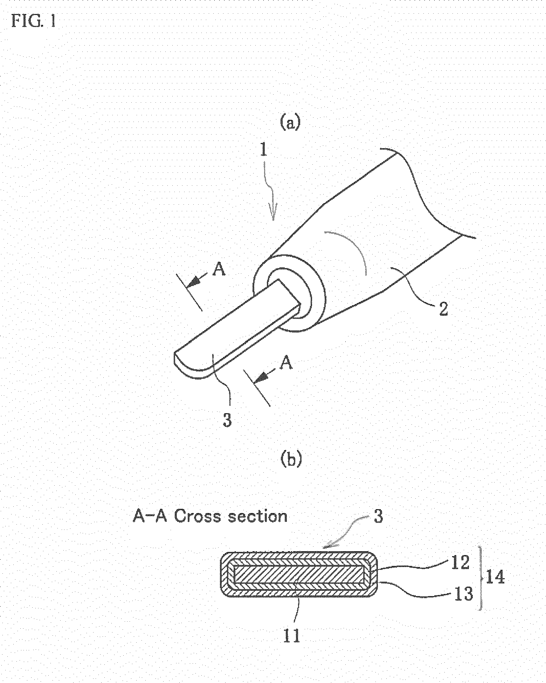

FIGS. 1(a) and (b) are diagrams illustrating a construction of an electrosurgical knife as an embodiment of the energy device for surgical operation according to the invention, respectively, wherein FIG. 1(a) is a perspective view of a part thereof and FIG. 1(b) is a sectional view taken along a line A-A in FIG. 1(a). The electrosurgical knife 1 shown in FIG. 1(a) as an example of the invention is constructed with a main body 2 of the electrosurgical knife and an operational area portion 3 protruded from the main body 2.

In actual use, the operational area portion 3 of the electrosurgical knife 1 is moved closer to a biological body to be processed (not shown) and microwave, radio-frequency wave or ultrasonic wave is transmitted from the operational area portion 3 to the biological body to perform incision of body tissue or coagulation of bleeding from the tissue (hemostasis) or both of incision and coagulation.

The feature of the invention lies in a construction of the operational area portion 3 shown in FIGS. 1(a) and (b). In the invention, the operational area portion 3 is constructed with a base material 11 and a coating layer 14 comprised of a base coating 12 disposed on an outer periphery of the base material 11 and an outermost coating 13 disposed on an outer periphery of the base coating 12 as shown in FIG. 1(b), wherein the base coating 12 constituting the coating layer 14 is made from silicon oxide or a compound containing silicon oxide and the outermost coating 13 constituting the coating layer 14 is made from polysiloxane or a compound containing polysiloxane or a compound containing a partly fluorinated polysiloxane. In such a construction, fixation of body tissue to the operational area portion can be suppressed by water-repellent and oil-repellent action of the outermost coating having a low surface energy. Also, since the base coating 12 strongly adheres to the base material 11 and the outermost coating 13, good adhesion property is obtained. Furthermore, the base coating 12 can be formed at room temperature or about 150°C, whereby the deformation of the base material 11 due to heat affection can be suppressed.

In the aforementioned embodiment, thickness of the base coating 12 is 0.1-1.0 µm. In this preferable embodiment, the base coating 12 can be formed uniformly in the base material 11 of a complicated form because it is a thin coating. Further, the outermost coating made from polysiloxane, a compound containing polysiloxane or a compound containing a partly fluorinated polysiloxane can prevent fixation of body tissue at a thickness of 0.1 µm. The thickness of the outermost coating 13 can be adjusted by application of plural times, but sufficient effects can be

In the above embodiment, a surface roughness of the base material 11 constituting the operational area portion 3 is preferably not more than 0.01-1.0 µm as Ra value before the formation of the coating layer 14. In this preferable example, the base material 11 can be adjusted to an arbitrary surface by electrolytic polishing, buffing, blast treatment with spherical or polygonal ceramic abrasive grains or laser irradiation to select surface form and roughness effective for suppressing fixation of body tissue. Also, the base material 11 constituting the operational area portion 3 is a metal, preferably stainless steel, titanium or titanium alloy, or a resin, preferably polyimide or PEEK resin. In this preferable example, it may be usually used as a material having a safeness to biological body and has a good adhesiveness to the base coating 12.

In the above embodiment, energy transmitted to the operational area portion 3 is preferable to be microwave, radio-frequency wave or ultrasonic wave. In this preferable embodiment, even if the energy used in the incision of body tissue, coagulation or hemostasis is any energy, the fixation of body tissue can be suppressed by forming the above coating layer 14. Also, the energy device for surgical operation can be used as a surgical knife, tweezers, forceps or snare used in the surgical operation. The energy device for surgical operation of this construction can be applied to the above product group by uniformly coating the whole periphery of the operational area portion with the coating layer suppressing the fixation of body tissue.

As a method of forming the base coating 12 to the base material 11, there can be used the conventionally known various methods. For example, flow coating, dipping, spraying or CVD method can be used.

As a method of forming the outermost coating 13 to the base material 11, there can be used the conventionally known various methods such as flow coating, dipping, spraying methods.

### EXAMPLES

There will be described examples in the energy device for surgical operation according to the above embodiments below.

### <Example 1>

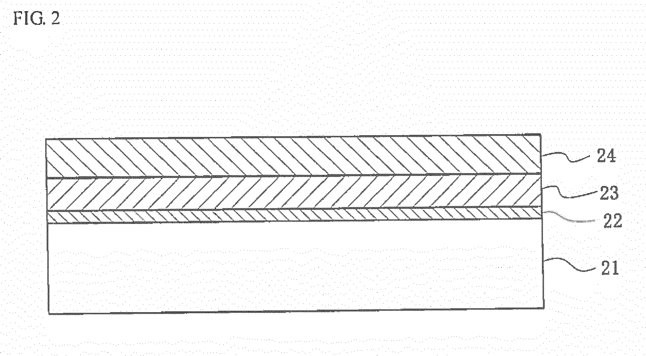

A test specimen having a coating layer shown in FIG. 2 is prepared as a simulation of an operational area portion 3 of an electrosurgical knife 1 as an energy device for surgical operation shown in FIGS. 1(a) and (b). In the example of FIG. 2, a surface of a base material 21 made of SUS 304 steel is subjected to a blast treatment to form a pretreated portion 22 having a surface roughness of Ra :1 µm. Then, a base coating 23 made of silicon oxide is formed from ethyl silicate (TEOS, for example, made by KOJUNDO Chemical Laboratory Co., Ltd.) at 100°C. Next, an outermost coating 24 made from a compound containing a fluorinated polysiloxane is formed on the base coating 23 by dipping. A test specimen of an invention example is prepared by the above process. As a test specimen of a comparative example are provided a test specimen not forming the base coating 23 and outermost coating 24, and a test specimen not forming the outermost coating 24 in the test specimen of the invention example. A contact angle of water and a contact angle of rapeseed oil are measured with respect to a surface of a coating layer in the test specimens of the invention example and comparative examples. The contact angle is measured by a sessile drop method according to JIS R3257. The results are shown in Table 1.

**Table 1**

| Base Material | Base coating | Outermost coating | Contact angle of water | Contact angle of rapeseed oil | Remarks |
|---|---|---|---|---|---|
| SUS304 steel | Absence | Absence | 35° | 10° | Comparative Example |
| | Presence | Absence | 15° | 8° | Comparative Example |
| | Presence | Presence | 110° | 70° | Invention Example |

As seen from the results of Table 1, the fixation of body tissue can be suppressed by water-repellent and oil-repellent actions of the outermost coating 24 having a low surface energy in the test specimen of the invention example as compared to the test specimens of the comparative examples.

### <Example 2>

There is examined a base coating in the coating layer according to the invention. After a surface of a base material made of SUS 304 steel with 25 mm (width) x 100 mm (length) x 1 mm (thickness) is subjected to a pretreatment according to Example 1, there are provided a test specimen of an invention example obtained by applying a base coating made of silicon oxide and forming an outermost coating made from a compound containing a fluorinated polysiloxane, a test specimen of an invention example obtained by forming a base coating made from an iron oxide through a heat treatment and then forming an outermost coating made from a compound containing a fluorinated polysiloxane, and a test specimen of a comparative example obtained by directly forming an outermost coating made from a compound containing a fluorinated polysiloxane. An evaluation of adhesiveness is performed by a rubbing test (ASTM D4752) with respect to the test specimens of the invention examples and the comparative example. The results are shown in Table 2.

**Table 2**

| Base material | Base coating | Outermost coating | Deformation of base material | Reciprocating number of base coating and base material exposed by rubbing test | Remarks |
|---|---|---|---|---|---|
| SUS304 steel | Absence | Compound containing a fluorinated polysiloxane 0.1 µm | Absence | 50 times | Comparative Example |
| | Silicon oxide | | Absence | 1000 times | Invention Example |
| | Iron oxide formed through heat treatment (400°C) | | Presence | 200 times | Comparative Example |

As seen from the results of Table 2, the test specimens of the invention examples having the base coating made of silicon oxide have many reciprocating numbers by rubbing test as compared to the test specimen of the comparative example forming no base coating and the test specimen of the comparative example forming iron oxide as a base coating through heat treatment and is high in the adhesiveness of the coating layer. Further, the test specimen of the comparative example forming iron oxide as the base coating through heat treatment causes the deformation in the formation of the base coating.

### <Example 3>

There is examined a preferable example of a thickness of a base coating in the coating layer according to the invention. After a surface of a base material made of rod SUS 304 steel with 2.7 mm (width) x 10 mm (length) is subjected to a pretreatment according to Example 1, there are provided test specimens obtained by forming a base coating made from silicon oxide while varying a thickness within a range of 0.01 µm to 15.0 µm and then forming an outermost coating made from a compound containing a fluorinated polysiloxane thereon. Each of the test specimens is inserted into a pig liver and microwave is introduced at 50 W for 30 seconds to evaluate a coagulation property of tissue and a burning degree. The results are shown in Table 3.

**Table 3**

| Base material | Base coating | Thickness of base coating (µm) | Outermost coating | Coagulation property | Resistance to burning | Remarks |
|---|---|---|---|---|---|---|
| SUS304 steel | Silicon oxide | 0.01 | Compound containing a fluorinated polysiloxane 0,1 µm | ○ | × | Comparative Example |
| | | 0.05 | | ○ | Δ | Invention Example |
| | | 0.1 | | ○ | ○ | Invention Example |
| | | 0.5 | | ○ | ○ | Invention Example |
| | | 1.0 | | ○ | ○ | Invention Example |
| | | 5.0 | | Δ | ○ | Invention Example |
| | | 10.0 | | Δ | ○ | Invention Example |
| | | 15.0 | | × | ○ | Invention Example |

| | | | | | | |
|---|---|---|---|---|---|---|
| Note 1) Coagulation property/ ○ coagulated at 50 W for 30 seconds Δ coagulated at 50 W for 45 seconds ○ not coagulated at 50 W for 60 seconds Note 2) Resistance to burning/ ○ no burning Δ removable by wiping × impossible to remove by wiping | | | | | | |

As seen from the results of Table 3, when the thickness of the base coating made of silicon oxide is less than 0.05 µm, the burning degree becomes larger, while when the thickness exceeds 10 µm, the burning is not caused, but the coagulation property of tissue becomes lower. To this end, the thickness of the base coating made of silicon oxide is preferable to be a range of 0.05-10.0 µm.

### <Example 4>

There is examined a preferable example of a pretreatment to the base material according to the invention. After any surface roughness Ra is applied to a surface of a base material made from a rod SUS 304 steel of 2.7 mm (diameter) x 10 mm (length) by electrolytic polishing, buffing, blast treatment with spherical or polygonal ceramic abrasive grains or laser irradiation, there are provided test specimens obtained by forming a base coating made of silicon oxide and an outermost coating made from a compound containing a fluorinated polysiloxane according to Example 1. Each of the test specimens is inserted into a pig liver and microwave is introduced at 50 W for 30 seconds to evaluate a a burning degree. The results are shown in Table 4.

**Table 4**

| Base material | Base coating | Outermost coating | Pretreatment | Surface roughness (Ra) | Resistance to burning | Remarks |
|---|---|---|---|---|---|---|
| | | | Absence | 0.1µm | ○ | Invention Example |
| | | | Electrolytic polishing | 0.01µm | ○ | Invention Example |
| | | | Buffing | 0.1 µm | ○ | Invention Example |
| | | | Blast treatment with spherical ceramic abrasive grains | 0.1µm | ○ | Invention Example |
| | | | | 0.5µm | ○ | Invention Example |
| | | | | 1.0µm | ○ | Invention Example |
| SUS304 steel | | Compound containing a fluorinated polysiloxane 0.1 µm | | 3.0µm | Δ | Invention Example |
| | Silicon oxide | | | 5.0µm | × | Comparative Example |
| | | | Blast treatment with polygonal ceramic abrasive grains | 0.1µm | ○ | Invention Example |
| | | | | 0.5µm | ○ | Invention Example |
| | | | | 1.0µm | ○ | Invention Example |
| | | | | 3.0µm | Δ | Invention Example |
| | | | | 5.0µm | × | Comparative Example |
| | | | Laser irradiation | 0.1µm | | Invention Example |
| | | | | 0.5µm | ○ | Invention Example |
| | | | | 1.0µm | ○ | Invention Example |
| | | | | 3.0µm | Δ | Invention Example |
| | | | | 5.0µm | × | Comparative Example |

| | | | | | | |
|---|---|---|---|---|---|---|
| Note) Resistance to burning/ ○ no burning Δ removable by wiping * impossible to remove by wiping | | | | | | |

As seen from the results of Table 4, when the surface roughness Ra of the base material is Ra: not more than 3.0 µm, further preferably Ra: 0.01-1.0 µm, the burning degree of liver tissue is particularly small and the fixation of body tissue can be suppressed. To this end, the surface roughness of the base material is preferable to be Ra: not more than 3.0 µm, and more preferable to be Ra: 0.01-1.0 µm.

### <Example 5>

There is examined a preferable kind of a base material. There are provided test specimens obtained by forming base coating made of silicon oxide and an outermost coating made from a compound containing a fluorinated polysiloxane on a base material with 25 mm (width) x 100 mm (length) x 1 mm (thickness) as shown in Table 5 according to Example 1. Each of the test specimens is heated to 100°C with a hot plate and a pig liver piece is placed thereon to evaluate a burning degree. The results are shown in Table 5.

**Table 5**

| Base material | Base coating | Outermost coating | Judgment of coating formation | Resistance to burning | Remarks |
|---|---|---|---|---|---|
| SUS304 steel | Silicon oxide | Compound containing a fluorinated polysiloxane 0.1 µm | ○ | ○ | Invention Example |
| SUS420J2 steel | | | ○ | ○ | Invention Example |
| SS400 steel | | | ○ | ○ | Invention Example |
| A5052 | | | ○ | ○ | Invention Example |
| A6061 | | | ○ | ○ | Invention Example |
| Pure titanium | | | ○ | ○ | Invention Example |
| Ti-6Al-4V | | | ○ | ○ | Invention Example |
| Polyimide | | | ○ | ○ | Invention Example |
| PEEK | | | ○ | ○ | Invention Example |
| Polycarbonate | | | ○ | ○ | Invention Example |

| | | | | | |
|---|---|---|---|---|---|
| Note ) Resistance to burning/ ○ no burning Δ removable by wiping × impossible to remove by wiping | | | | | |

As seen from the results of Table 5, the base coating and outermost coating can be formed irrespectively of the kind of the base material, and the fixation of body tissue can be suppressed. Moreover, stainless steel, titanium or titanium alloy and polyimide, PEEK resin are suitable in consideration with safeness to biological body.

### <Example 6>

There is examined a preferable example of energy supplied to the operational area portion according to the invention. As an operational area portion of an energy device for surgical operation using microwave, radio-frequency wave, ultrasonic wave or electric energy, a coating layer comprised of a base coating made of silicon oxide and an outermost coating made from a compound containing a fluorinated polysiloxane is formed on a base material and then incision and coagulation of a pig liver are performed to evaluate a burning degree. The results are shown in Table 6.

**Table 6**

| Base material | Base coating | Outermost coating Energy | | Resistance to burning | Remarks |
|---|---|---|---|---|---|
| SUS304 steel | Silicon oxide | Compound containing a fluorinated polysiloxane 0.1 µm | Microwave | ○ | Invention Example |
| | | | Radio-frequency wave | ○ | Invention Example |
| | | | Ultrasonic wave | ○ | Invention Example |

| | | | | | |
|---|---|---|---|---|---|
| Note ) Resistance to burning/ no burning Δ removable by wiping × impossible to remove by wiping | | | | | |

As seen from the results of Table 6, the fixation of body tissue can be suppressed by forming the base coating made of silicon oxide and the outermost coating made from a compound containing a fluorinated polysiloxane even in any energy devices.

### <Example 7>

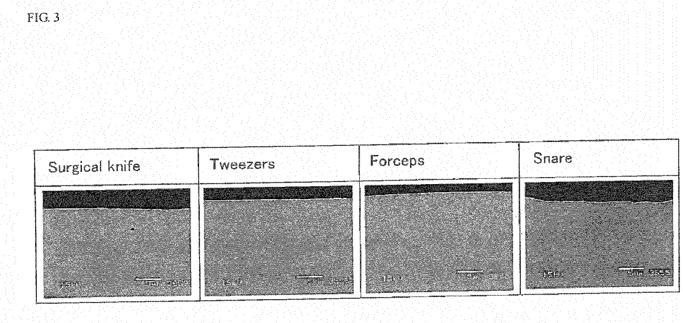

A base coating made of silicon oxide and an outermost coating made from a compound containing a fluorinated polysiloxane are formed on a base material of an operational area portion of a surgical knife, tweezers, forceps or snare used in the surgical operation and thereafter a section structure of each of the coatings is observed to obtain results shown in FIG. 3, As seen from the results of FIG. 3, the base coating made of silicon oxide and the outermost coating made from a compound containing a fluorinated polysiloxane can be formed irrespectively of the form of the operational area portion in the energy device for surgical operation.

Although silicon oxide is used as the base coating in the above examples, results similar to those of the above examples can be obtained even if a mixture containing silicon oxide is used instead of silicon oxide as the base coating. Also, although the compound containing a fluorinated polysiloxane is used as the outermost coating, results similar to those of the above examples can be obtained even if polysiloxane or a mixture containing polysiloxane is used instead of the compound containing a fluorinated polysiloxane.

### INDUSTRIAL APPLICABILITY

According to the invention, there can be provided an energy device for surgical operation with coating capable of suppressing fixation of body tissue in an operational area portion of the surgical energy device, which can be preferably used as a surgical knife, tweezers, forceps or snare.

### DESCRIPTION OF REFERENCE SYMBOLS

- 1: electrosurgical knife
- 2: main body of electrosurgical knife
- 3: operational area portion
- 11, 21: base material
- 12, 23: base coating
- 13, 24: outermost coating
- 14: coating layer
- 22: pretreated portion

## Claims

1. An energy device for surgical operation having an operational area portion (3) for transmitting energy to a body target portion, **characterized in that** a coating layer (14) formed on an outer periphery of a base material (11) constituting the operational area portion (3) is comprised of a base coating (12) having a thickness of 0.1-1.0 µm formed on the base material (11) and an outermost coating (13) having a thickness of 0.1 µm formed on the base coating (12), the base coating (12) is silicon oxide or a mixture containing silicon oxide, while the outermost coating (13) is polysiloxane or a compound containing polysiloxane or a compound containing a partly fluorinated polysiloxane, and wherein a surface roughness of the base material (11) is 0.01-1.0 µm as Ra value before the formation of the coating layer (14).

2. The energy device for surgical operation according to claim 1, wherein energy transmitted from the operational area portion (3) to biological tissue is microwave, radio-frequency wave or ultrasonic wave.

3. The energy device for surgical operation according to any one of claims 1 to 2, wherein the energy device for surgical operation is a surgical knife, tweezers, forceps or snare.

## Patentansprüche

1. Energievorrichtung für chirurgische Eingriffe mit einem Operationsbereich-Abschnitt (3) zum Übertragen von Energie zu einem Körper-Zielabschnitt, **dadurch gekennzeichnet, dass** eine Überzugsschicht (14), die an einem Außenumfang eines Trägermaterials (11) ausgebildet ist, das den Operationsbereich-Abschnitt (3) bildet, aus einem Basis-Überzug (12) mit einer Dicke von 0,1 - 1,0 µm, der auf dem Trägermaterial (11) ausgebildet ist, und einem Außen-Überzug (13) mit einer Dicke von 0,1 µm besteht, der auf dem Basis-Überzug (12) ausgebildet ist, wobei der Basis-Überzug (12) Siliziumoxid oder ein Siliziumoxid enthaltendes Gemisch ist, während der Außen-Überzug (13) Polysiloxan oder eine Polysiloxan enthaltende Verbindung oder eine ein teilweise fluoriertes Polysiloxan enthaltende Verbindung ist, und wobei eine Oberflächenrauigkeit des Trägermaterials (11) vor der Ausbildung der Überzugsschicht (14) 0,01 - 1,0 µm als Ra-Wert beträgt.

2. Energievorrichtung für chirurgische Eingriffe nach Anspruch 1, wobei die vom Operationsbereich-Abschnitt (3) auf biologisches Gewebe übertragene Energie Mikrowellen-Energie, Hochfrequenzwellen-Energie oder Ultraschallwellen-Energie ist.

3. Energievorrichtung für chirurgische Eingriffe nach einem der Ansprüche 1 bis 2, wobei die Energievorrichtung für chirurgische Eingriffe ein chirurgisches Messer, eine Pinzette, eine Zange oder eine Schlinge ist.

## Revendications

1. Dispositif énergétique pour opération chirurgicale ayant une portion de surface opérationnelle (3) pour transmettre de l'énergie à une portion corporelle cible, **caractérisé en ce qu'**une couche de revêtement (14) formée sur une périphérie externe d'un matériau de base (11) constituant la portion de surface opérationnelle (3) est constituée d'un revêtement de base (12) ayant une épaisseur de 0,1 à 1,0 µm formé sur le matériau de base (11) et d'un revêtement le plus externe (13) ayant une épaisseur de 0,1 µm formé sur le revêtement de base (12), le revêtement de base (12) étant de l'oxyde de silicium ou un mélange contenant de l'oxyde de silicium, tandis que le revêtement le plus externe (13) est le polysiloxane ou un composé contenant du polysiloxane ou un composé contenant un polysiloxane partiellement fluoré, et une rugosité de surface du matériau de base (11) étant de 0,01 à 1,0 µm comme valeur Ra avant la formation de la couche de revêtement (14).

2. Dispositif énergétique pour opération chirurgicale selon la revendication 1,
l'énergie transmise depuis la portion de surface opérationnelle (3) au tissu biologique étant des micro-ondes, une onde de radiofréquence ou une onde ultrasonore.

3. Dispositif énergétique pour opération chirurgicale selon l'une quelconque des revendications 1 à 2,
le dispositif énergétique pour opération chirurgicale étant un couteau chirurgical, des pincettes, des forceps ou une anse.
